# EUROPEAN PATENT APPLICATION

(11) **EP 4 230 154 A1**
(43) Date of publication of application: **23.08.2023**
(21) Application number: 20957198.3
(22) Date of filing: 16.10.2020
(51) Int. Cl.: A61B 17/04

(54) **TYING STRUCTURE CAPABLE OF BEING USED FOR SUTURING**

(71) Applicant: Matrixlabs Medical Co., Ltd., New Taipei, Taiwan 244 (TW)
(72) Inventor: TU, Fung-Chao, New Taipei, Taiwan 22060 (TW); WU, Wen-Yih, Taipei, Taiwan 10550 (TW); CHEN, Kuei-Hua, Kaohsiung, Taiwan 83159 (TW)
(74) Representative: Cabinet Chaillot
(86) International application number: PCT/CN2020/121414
(87) International publication number: WO 2022/077420

(57) **Abstract**

A knotting assembly for tying a surgical knot has an outer tube (10) and an inner tube (11) being slidably mounted in the outer tube (10). The inner tube (11) protrudes from the outer tube (10) and forms a neck (111) between a distal end thereof and a distal end of the outer tube (10). A suture material (20) is looped surround the neck (111) to form a pre-tied knot (21) and a loop (22). The suture material (20) has an inner section being moveably inserted in the inner tube (11). The loop (22) is inserted by at least one strand of suture filament. While the inner section is pulled, the loop (22) is closed to hold the suture filament (30) and folds the suture filament (30) and pulls the folded suture filament (30) into the inner tube to finish a surgical knot (A).

## Description

### FIELD

The present disclosure relates to a surgical knot assembly adapted for facilitating the process of suturing and knot-tying in a surgical operation for an operator.

### BACKGROUND

Surgical suturing typically requires a well knotted end to hold and maintain a body tissue or a treatment site together to stop bleeding and to promote healing.

However, conventional knot-tying process and techniques can be exceedingly difficult, complex and time-consuming. Therefore, there is a need to improve the conventional knot-tying process and approach.

### SUMMARY OF THE DISCLOSURE

To solve the aforementioned problems, the main objective of the present disclosure is to provide a surgical knot assembly and methods of use thereof that is simple and time-saving for assisting healthcare professionals at delivering a secure surgical knot with consistent knot strength each time.

To achieve the said objectives, the present disclosure provides a surgical knot assembly for tying a surgical knot, comprising: an outer tube; an inner tube slidably interconnected within the outer tube and having a distal end protruding from the outer tube as a neck; and a strand of suture material having a first portion of a length configured releasably engaged to the neck being a pre-tied knot, and having a second portion of a length extending from a cavity at the distal end of the inner tube, thereby forming a loop between the distal end of the inner tube and the neck to receive at least a strand of suture filament therein, wherein size of the loop is variable by manoeuvring the suture material and closing the loop as the neck retracts back into the outer tube, allowing the loop, the pre-tied knot and the suture filament to be fastened together creating a surgical knot held at the cavity.

Preferably, the cavity of the inner tube has a smaller diameter than a sum of a diameter of the loop, the pre-tied knot and the suture filament fastened together.

Preferably, the pre-tied knot is whirled around the inner tube for at least twice, wherein the suture material and at least a strand of the suture filament having a same measurement and dimension.

Preferably, at least a strand of the suture filament having at least two segments intertwining with the loop.

Preferably, the suture filament first penetrates through a body tissue or a treatment site before intertwining with the loop.

Preferably, when the assembly in use and the loop has received at least a strand of the suture filament therein readily for tightening or fastening to create a secure surgical knot, size of the loop is variable by manoeuvring the suture material from a control of the assembly. As the loop tightens, the neck retracts back into the outer tube, allowing the loop, the pre-tied knot and the suture filament to be fastened together and thereby creating a surgical knot held at the cavity.

### BRIEF DESCRIPTION OF THE DRAWINGS

Figure 1 is a perspective view of a first embodiment of a surgical knot assembly in accordance with the present disclosure.
Figures 2 to 4 depicts the surgical knot assembly of Figure 1 in operation.
Figure 5 is a perspective view of a second embodiment of a surgical knot assembly in accordance with the present disclosure.
Figures 6 to 8 depicts the surgical knot assembly of Figure 5 in operation.
Figure 9 is a perspective view of a third embodiment of a surgical knot assembly in accordance with the present disclosure.
Figure 10 is a perspective view of a fourth embodiment of a surgical knot assembly in accordance with the present disclosure.

### DETAILED DESCRIPTION OF THE DISCLOSURE

Other objectives, advantages and novel features of the present disclosure will become more apparent from the following detailed description when taken in conjunction with the accompanying drawings.

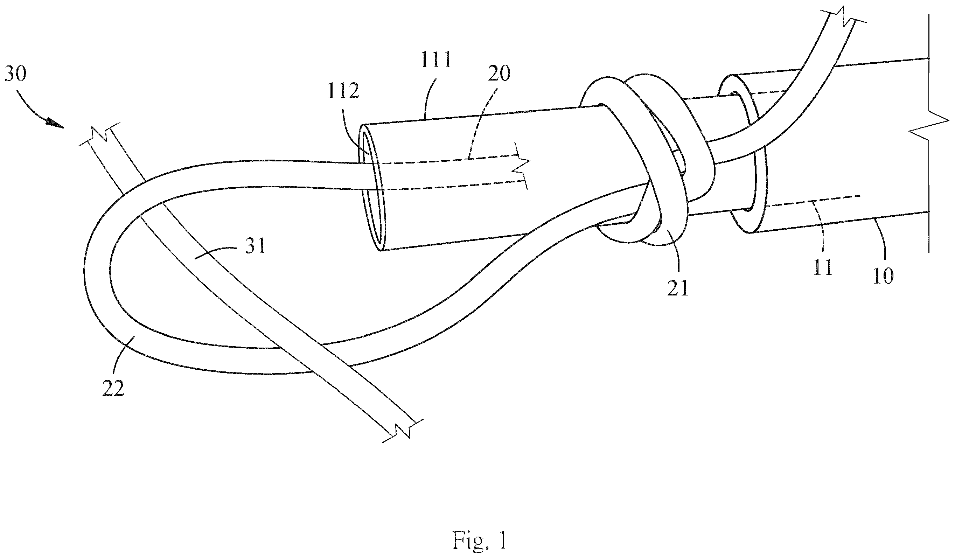

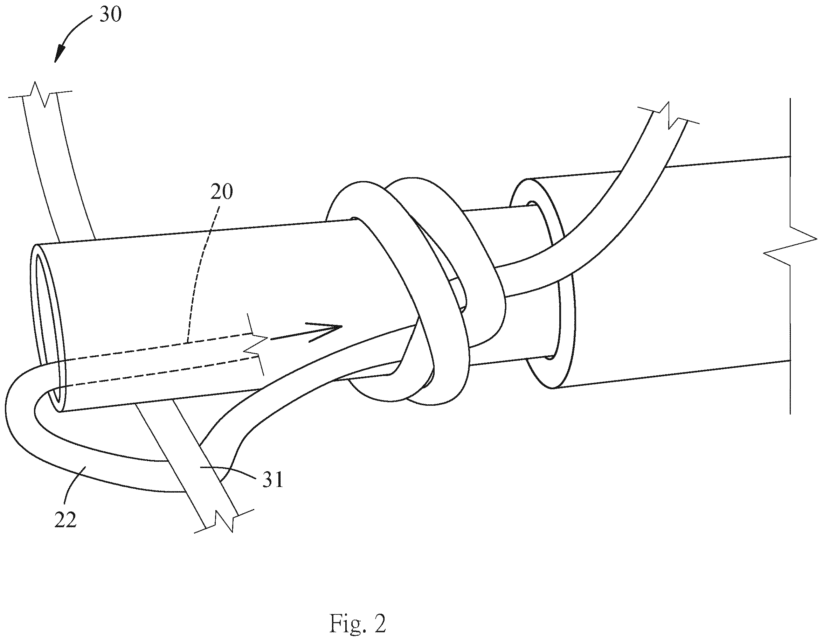

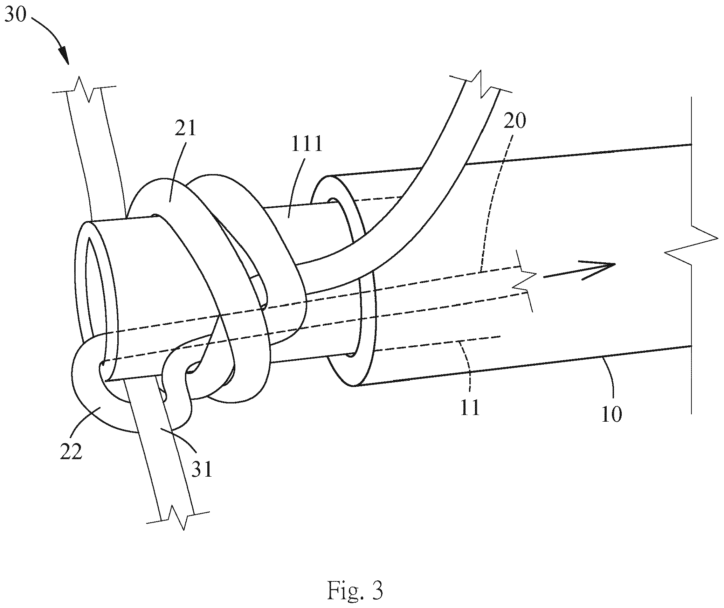

With reference to Figs. 1 to 4, according to a preferred embodiment of the present disclosure, a surgical knot assembly for tying a surgical knot is shown. The surgical knot assembly for tying a surgical knot comprises an outer tube 10, an inner tube 11 slidably interconnected within the outer tube 10 and having a distal end protruding from the outer tube 10 as a neck 111, and a strand of suture material 20 having a first portion of a length configured releasably engaged to the neck 111 being a pre-tied knot 21, and having a second portion of a length extending from a cavity 112 at the distal end of the inner tube 11, thereby forming a loop 22 between the distal end of the inner tube 11 and the neck 111 The pre-tied knot 21 can be configured and/or arranged to accommodate a particular desired strength of knot. Preferably, the pre-tied knot 21 is whirled around the inner tube 11 for at least once. With reference to the present embodiment, a two-turned slip knot is shown.

The loop 22, formed between the distal end of the inner tube 11 and the neck 111, receives at least a strand of suture filament 30 therein, and the size of the loop 22 is variable by manoeuvring the suture material 20 and closing the loop 22 as the neck 111 retracts into the outer tube 10, allowing the loop 22, the pre-tied knot 21 and the suture filament 30 to be fastened together creating a surgical knot A held at the cavity 112. The suture filament 30 has a knotted segment 31 where may be formed with the surgical knot A. The knotted segment 31 extends through the loop 22, and the inner section of the first suture material 20 is pulled. With a rear movement of the first suture material 20, the loop 22 is closed and abuts against the distal end of the inner tube 11 and the inner tube 11 is moved into the outer tube 10. At the same time, the loop 22 is moved over the distal ends of the outer tube 10 and the inner tube 11 and across the knotted segment 31, such that the knotted segment 31 is folded to form a folded segment 311 on the knotted segment 31. The folded segment 311 is moved into the distal end of the cavity 112. In addition, the pre-tied knot 21 is removed from the neck 111 and is tied around the folded segment 311 of the knotted segment 31 to form the surgical knot A. Finally, the front end of the first suture material 20 or the suture filament 30 can be cut off. The suture material 20 can be manoeuvred via a control of the assembly. In certain embodiments, the diameters of the suture material 20 and suture filament 30 have the same and/or close diameter measurements to ensure the efficiency, robustness, and security of the surgical knot.

Because the folded segment 311 on the knotted segment 31 is moved into the distal end of the cavity 112 before forming the surgical knot A, the distal end of the cavity 112 has a diameter being larger than a sum of a diameter of the first suture material 20 and a width of the folded segment 311. In some embodiments, the dimensions and/or specification of the suture material 20 and suture filament 30 are vastly different. Therefore, as the surgical knot assembly is readily detachable from each other, the operator can replace parts of the assembly and/or choose and apply the applicable configuration thereof as required.

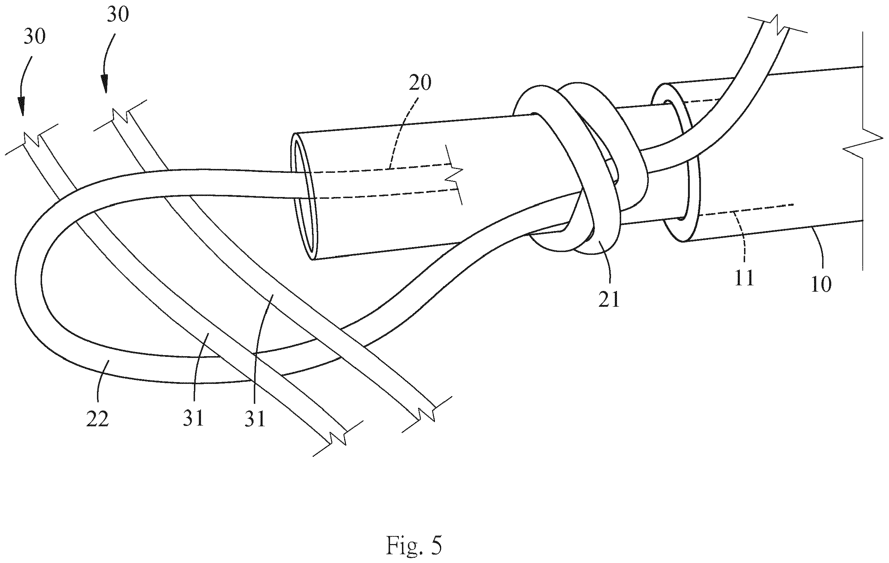

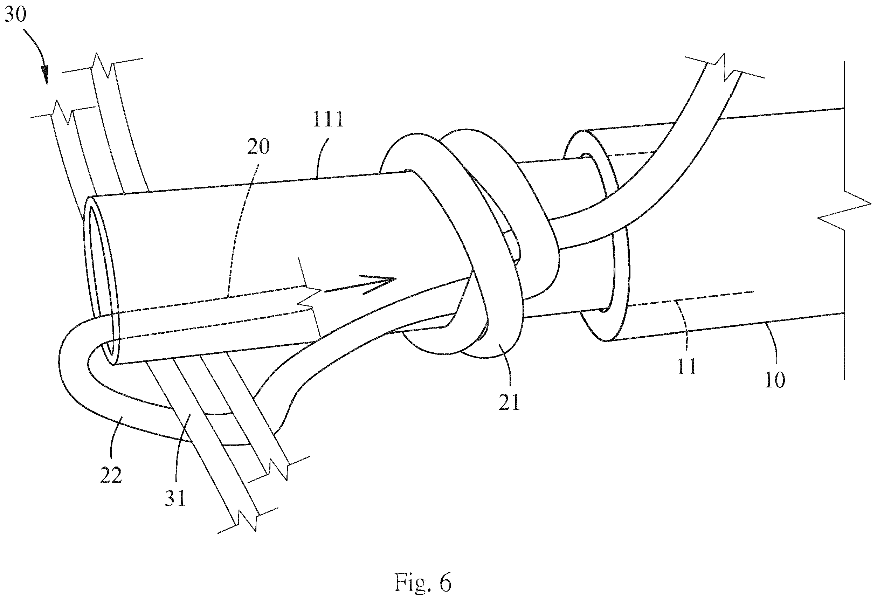

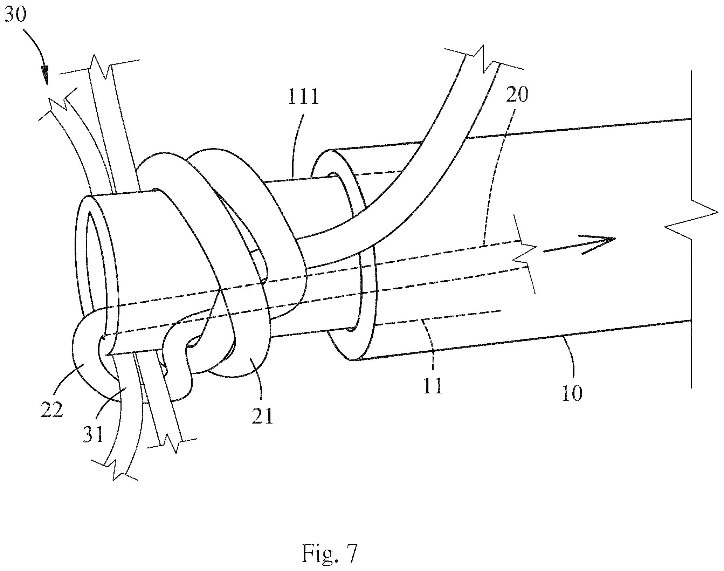

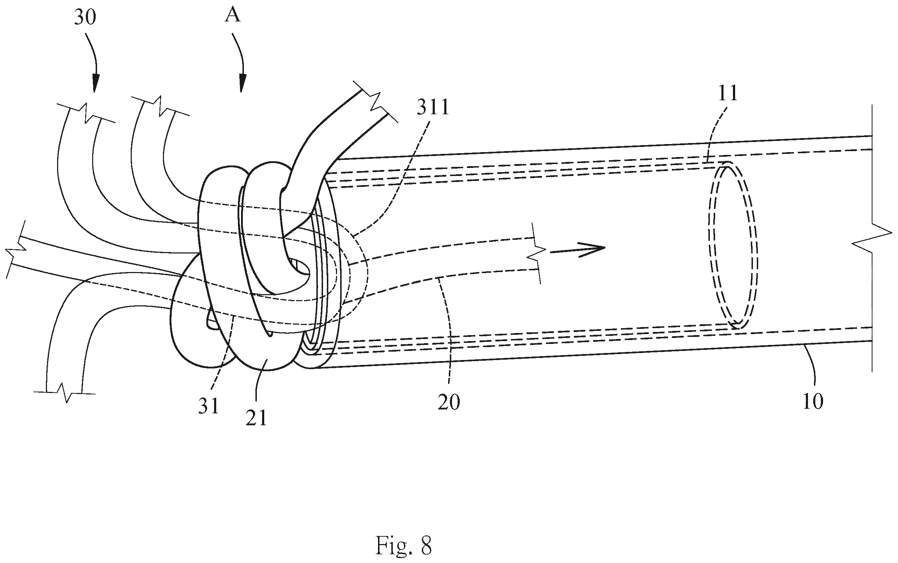

Referring to Figs. 5 -8, in one of the preferred embodiments, the suture material 20 is manoeuvred via the control as to close the loop 22. The loop 22 receives two strands of suture filament 30 therein and fastened to create the surgical knot A. As the surgical knot A is tightly fastened, the overall widths of the suture material 20 and filaments 30 are reduced and further compressed for knot security engaging the cavity 112.

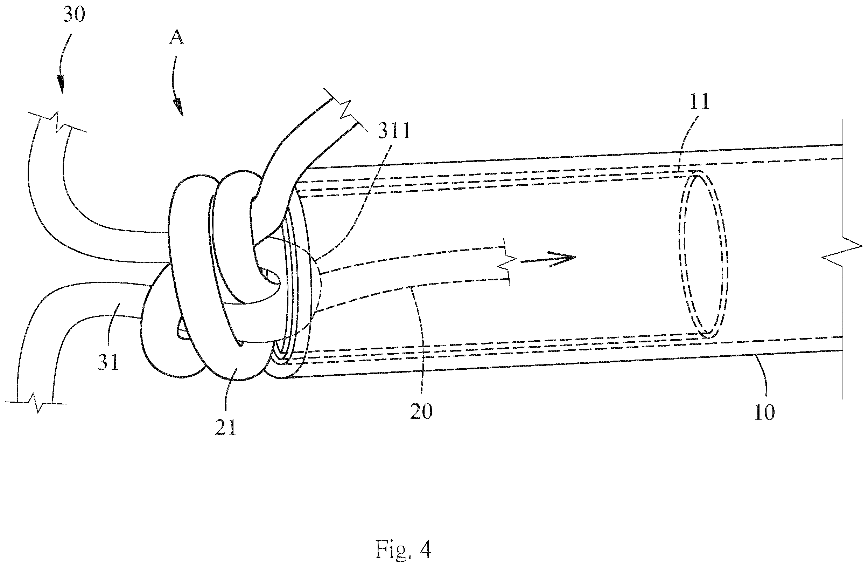

The relative position between the outer tube 10, the inner tube 11 and the pre-tied knot 21 is variable subject to the working and fastening of the suture filament 30 and can be fixed during the operation by the operator to ensure that the suturing and knot-tying process for a patient can be adequately tended. As the operator retracts the inner tube 11 and tightens the loop 22, the surgical knot A is partially created and identified at the tip of the cavity 112. Preferably, the cavity 112 has a smaller diameter measurement than the suture material 20, suture filament 30 and pre-tied knot fastened together, e.g., the partially created surgical knot A has a greater diameter in width than the cavity 112, as shown in Fig. 4. In some embodiments, the cavity 112 provides holding and limitation of movements for the partially created surgical knot A to prevent disengagement thereof before the surgical knot A is fully tightened.

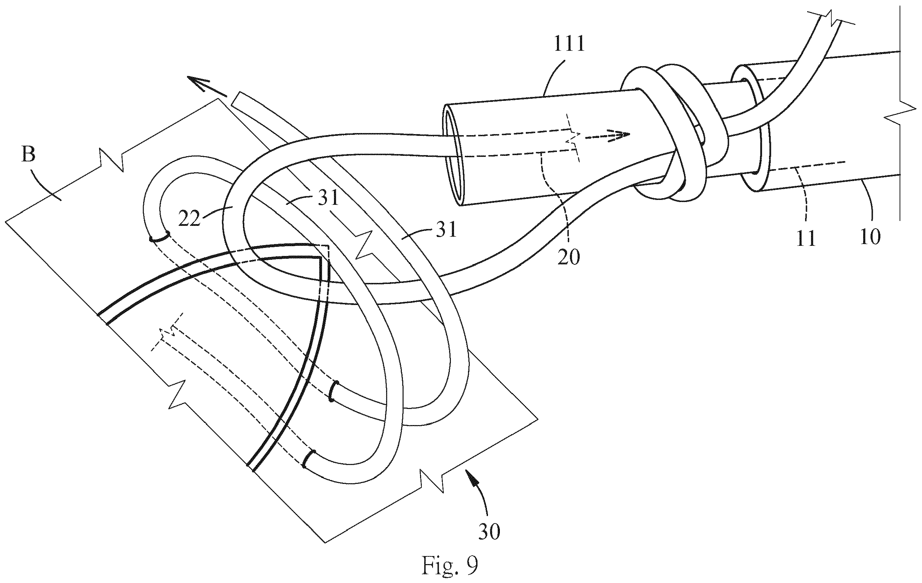

In one embodiment, the suture filament 30 has a plurality of segments intertwined with the loop 22 before forming the surgical knot to obtain desired knot strength as shown in Figure 9.

In some embodiments, the number of times that the pre-tied knot 21 is whirled around the inner tube 11 is subject to the suture material 20 used, number of strands of the filament 30 applied and the desired knot strength that the operator sees fit for the operation. There could be several ways of how the pre-tied knot is configured in conjunction with the usage of the assembly. A maximum compression capacity of the suture material 20 and filament 30 is pre-determined with the selected combination of the surgical knot assembly. The operator selects and/or choose the preferred configuration of the assembly, then a corresponding pre-determined point as to how far the partially formed surgical knot could reach within inner tube 11 is set, such that once reached it would not slide any further into the cavity 112. The operator would perceive the tactile sense of tying the knot as tightened in light of the friction and resistance starts to develop and the knot abutting to the walls of the cavity 112 of the inner tube 11 tightly. This mechanism ensures the tightness of the created surgical knot is solid, but not jammed in the cavity 112 and is readily available for placement at the treatment site B.

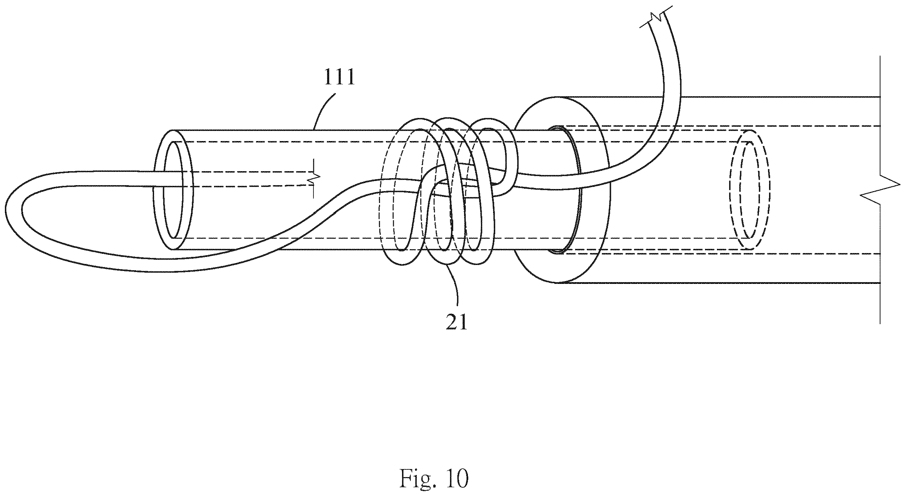

In some embodiments, the pre-tied knot 21 is whirled multiple times around the inner tube 11, as shown in Figure 10. Although there are multiple different embodiments of the surgical knot assembly, but each of the applicable configuration and/or arrangement has its own merits to accommodate the relevant fittings for the operation.

Even though numerous characteristics and advantages of the present disclosure have been set forth in the foregoing description, together with details of the structures and functions thereof, the present disclosure is illustrative only, and changes may be made in detail, especially in matters of shape, size, and arrangement of parts within the principles of the disclosure to the full extent indicated by the broad general meaning of the terms in which the appended claims are expressed.

### REFERENCED NUMBER LIST

| | |
|---|---|
| 10 outer tube | 11 inner tube |
| 111 neck | 112 cavity |
| 20 suture material | 21 pre-tied knot |
| 22 loop | 30 suture filament |
| 31 knotted segment | 311 folded segment |
| A surgical knot | B treatment site |

## Claims

1. A surgical knot assembly for tying a surgical knot, comprising:
an outer tube (10);
an inner tube (11) slidably interconnected within the outer tube (10) and having a distal end protruding from the outer tube (10) as a neck (111); and
a strand of suture material (20) having a first portion of a length configured releasably engaged to the neck (111) being a pre-tied knot (21), and having a second portion of a length extending from a cavity (112) at the distal end of the inner tube (11), thereby forming a loop (22) between the distal end of the inner tube (11) and the neck (111), at least a strand of suture filament (30) extends through the loop (22),
wherein size of the loop (22) is variable by manoeuvring the suture material (20) and closing the loop (22) as the neck (111) retracts back into the outer tube (10), allowing the loop (22), the pre-tied knot (21) and the suture filament (30) to be fastened together creating a surgical knot held at the cavity (112).

2. The surgical knot assembly according to claim 1, wherein the cavity (112) of the inner tube (11) has a smaller diameter than a sum of a diameter of the loop (22), the pre-tied knot (21) and the suture filament (30) fastened together.

3. The surgical knot assembly according to claim 2, wherein the pre-tied knot (21) is whirled around the inner tube (11) for at least one time and the suture material (20) and the suture filament (30) have a same measurement and dimension.

4. The surgical knot assembly according to claim 1, wherein the suture filament (30) has a plurality of segments intertwining with the loop (22).

5. The surgical knot assembly according to claim 4, wherein the suture filament (30) first penetrates through a body tissue or a treatment site before intertwining with the loop (22).

6. The surgical knot assembly according to claim 4, wherein the suture material (20) is partially secured on the outer tube (10).

7. The surgical knot assembly according to claim 2, wherein two strands of the suture filaments (30) intertwining with the loop (22).

8. The surgical knot assembly according to claim 3, wherein two strands of the suture filaments (30) have plurality of segments intertwining with the loop (22).

9. The surgical knot assembly according to claim 7, wherein the suture filament (30) first penetrates through a body tissue or a treatment site before intertwining with the loop (22).

10. The surgical knot assembly according to claim 8, wherein the suture filament (30) first penetrates through a body tissue or a treatment site before intertwining with the loop (22) and the suture material (20) is partially secured on the outer tube (10).
